# EUROPÄISCHE PATENTANMELDUNG

(11) **EP 2 385 033 A1**
(43) Veröffentlichungstag der Anmeldung: **09.11.2011**
(21) Anmeldenummer: 10162077.1
(22) Anmeldetag: 06.05.2010
(51) Int. Cl.: C07C 67/313, C07C 69/34

(54) **Verfahren zur oxidativen Spaltung funktionalisierter Cycloalkenderivate**

(71) Anmelder: Bayer CropScience AG, 40789 Monheim (DE)
(72) Erfinder: Schmidt, Jan, Peter, Dr., 65719 Hofheim (DE); Ford, Mark, James, Dr., 61389 Schmitten (DE)

(57) **Zusammenfassung**

Die vorliegende Erfindung betrifft ein Verfahren zur Herstellung funktionalisierter Omega-dicarbonsäuren.

## Beschreibung

Die vorliegende Erfindung betrifft ein Verfahren zur Herstellung von Omega-dicarbonsäuren.

Omega-dicarbonsäuren der Formel (I) sind wichtige Intermediate zur Herstellung von Pflanzenschutzmitteln, wie beispielsweise aus WO 95/07022 bekannt.

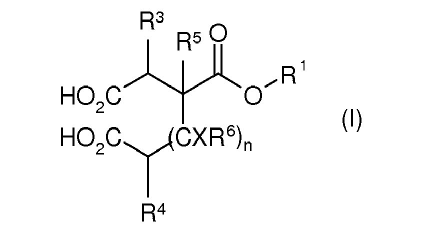

Aus WO 95/07022 ist auch bekannt, dass die Synthese solcher Derivate in brauchbaren Ausbeuten und Reinheiten der Nutzung von Kaliumpermanganat bedarf. Dabei werden jedoch große Mengen an toxischen Abfällen erzeugt, die die Anwendung in großtechnischem Maßstab unattraktiv gestalten.

Alternative Verfahren zur oxidativen Spaltung von Cycloalkenderivaten beinhalten die großtechnisch genutzte Umsetzung mit Salpetersäure, Einsatz von Rutheniumtetroxid bzw. Osmiumtetroxid in Verbindung mit einem stoichiometrischen Oxidationsmittel (JP 2005/086045, US 2003/0149299) und die Ozonolyse mit oxidativer Aufarbeitung (EP-A-0513600). Die industriell genutzte Spaltung mit Salpetersäure führt jedoch unweigerlich zur Entstehung von äquimolaren Mengen des Treibhausgases Distickstoffmonoxid, welches weiterer Entsorgung bedarf. Die katalytischen Osmium-/ und Rutheniumsysteme erzeugen ebenso äquimolare Mengen an giftigen Abfallprodukten von den stoichiometrischen Oxidanten während die Ozonolyse technisch schwer zu realisieren ist.

Eine weitere, beispielsweise aus WO 2009/109857 und US 5,047,582 bekannte Methode zur oxidativen Spaltung von Cycloalkenderivaten, ist die Nutzung von Wasserstoffperoxid in der Gegenwart von Natriumwolframat bzw. Wolframsäure mittels Phasentransferkatalyse. Die beschriebenen Reaktionsbedingungen erlauben es jedoch nicht, dass Produkte der allgemeinen Formel (I) erzeugt werden, da unter den sauren Bedingungen ein hohes Maß an Esterverseifung stattfindet. Es ist literaturbekannt, dass die oxidative Spaltungen von Olefinen zu Dicarbonsäuren mit Wasserstoffperoxid in der Gegenwart von Wolframsäure am besten ablaufen, wenn der pH des Reaktionsmediums zwischen pH 4 und pH 5 liegt (Oguchi, T.; Ura, T.; Ishii, Y.; Ogawa, M. Chem. Lett. 1989, 857). Zudem zeigte sich, dass die Reaktivität des Wolframkatalysators durch die Wahl des Liganden beeinflusst werden kann, sobald auf Phasentransferkatalyse verzichtet wird (Jiang, H.; Gong, H.; Yang, Z.; Zhang, X.; Sun, Z. React. Kinet. Catal. Lett. 2002, 75, 315). Ferner wurde von Jian *et al.* gezeigt, dass, für sich genommen, basische und neutrale Liganden die oxidative Spaltung unterdrücken und einige Carbonsäuren, wie zum Beispiel Essigsäure, Katalyseinhibitoren sind.

Aufgabe der vorliegenden Erfindung ist die Bereitstellung eines neuen Verfahrens zur Herstellung von Omega-dicarbonsäuren der Formel (I), das die Nachteile der bekannten Verfahren nicht aufweist, für die großtechnische Durchführung geeignet ist und Omega-dicarbonsäuren in hoher Ausbeute und Reinheit bei optimaler Katalysatorproduktivität liefert.

Es wurde nun gefunden, dass sich Verbindungen der Formel (II) mit Wasserstoffperoxid in wässrigen Carbonsäuren in Anwesenheit eines Katalysators der Formel (III) zu Omega-dicarbonsäuren der Formel (I) umsetzen lassen.

Das erfindungsgemäße Verfahren zur Herstellung von Verbindungen der Formel (I) kann durch folgendes Schema veranschaulicht werden:

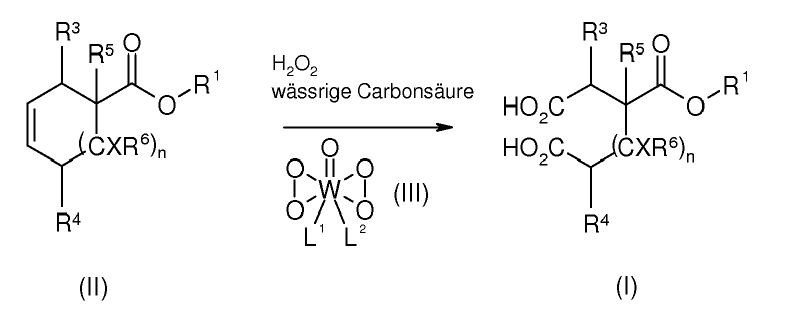

In den Formeln (I) und (II) stehen
X für COOR²,
R¹ für C₁-C₈-Alkyl, C₃-C₈-Cycloalkyl oder Benzyl,
R² für C₁-C₈-Alkyl, C₃-C₈-Cycloalkyl oder Benzyl,
R³ und R⁴ gleich oder verschieden für Wasserstoff, C₁-C₈-Alky oder C₁-C₈-Alkoxy,
R⁵ und R⁶ gleich oder verschieden für Wasserstoff oder C₁-C₈-Alky,
R³ und R⁵ oder/und R³ und R⁶ oder/und R³ und R⁴ oder/und R⁵ und R⁶ oder/und R⁵ und R⁴ oder/und R⁶ und R⁴ gemeinsam mit den Kohlenstoffatomen, an die sie gebunden sind, für gegebenenfalls durch
Halogen, C₁-C₆-Alkyl, C₁-C₆-Alkoxy oder C₁-C₆-Halogenalkyl substituiertes C₃-C₅-Alkandiyl, wobei ein oder mehrere Cyclen gebildet werden können,
n für 0 oder 1;
R¹ bevorzugt für C₁-C₄-Alkyl, Cyclopropyl, Cyclobutyl, Cyclopentyl, Cyclohexyl oder Benzyl,
R² bevorzugt für C₁-C₄-Alkyl, Cyclopropyl, Cyclobutyl, Cyclopentyl, Cyclohexyl oder Benzyl,
R³ und R⁴ gleich oder verschieden bevorzugt für Wasserstoff, C₁-C₄-Alkyl oder C₁-C₄-Alkoxy,
R⁵ und R⁶ gleich oder verschieden bevorzugt für Wasserstoff oder C₁-C₄-Alkyl,
R³ und R⁵ oder/und R³ und R⁶ oder/und R³ und R⁴ oder/und R⁵ und R⁶ oder/und R⁵ und R⁴ oder/und R⁶ und R⁴ gemeinsam mit den Kohlenstoffatomen, an die sie gebunden sind, bevorzugt für gegebenenfalls durch Halogen, C₁-C₄-Alkyl, C₁-C₄-Alkoxy oder C₁-C₄-Halogenalkyl substituiertes C₃-C₄-Alkan-diyl, wobei ein oder zwei Cyclen gebildet werden können,
n für 0 oder 1;
R¹ besonders bevorzugt für Methyl, Ethyl, n-Propyl, i-Propyl, n-Butyl, i-Butyl, t-Butyl oder Benzyl,
R² besonders bevorzugt für Methyl, Ethyl, n-Propyl, i-Propyl, n-Butyl, i-Butyl, t-Butyl oder Benzyl,
R³ und R⁴ gleich oder verschieden besonders bevorzugt für Wasserstoff, Methyl, Ethyl, n-Propyl, i-Propyl, n-Butyl, i-Butyl, t-Butyl, Methoxy, Ethoxy, n-Propoxy, i-Propoxy, n-Butoxy, i-Butoxy oder t-Butoxy,
R⁵ und R⁶ gleich oder verschieden besonders bevorzugt für Wasserstoff, Methyl, Ethyl, n-Propyl, i-Propyl, n-Butyl, i-Butyl oder t-Butyl,
R³ und R⁵ oder R³ und R⁶ oder R³ und R⁴ oder R⁵ und R⁶ oder R⁵ und R⁴ oder R⁶ und R⁴ gemeinsam mit den Kohlenstoffatomen, an die sie gebunden sind, besonders bevorzugt für gegebenenfalls durch Fluor, Chlor, Methyl, Ethyl, n-Propyl, iso-Propyl, Methoxy, Ethoxy, n-Propoxy, iso-Propoxy oder Trifluormethyl substituiertes C₄-Alkandiyl,
n für 0 oder 1;
R¹ ganz besonders bevorzugt für Benzyl,
R² besonders bevorzugt für Ethyl,
R³, R⁴, R⁵ und R⁶ ganz besonders bevorzugt für Wasserstoff,
n ganz besonders bevorzugt für 1;
R¹ ebenfalls ganz besonders bevorzugt für Benzyl,
R³, R⁴ und R⁵ ebenfalls ganz besonders bevorzugt für Wasserstoff,
n ebenfalls ganz besonders bevorzugt für 0.

Die oben aufgeführten allgemeinen oder in Vorzugsbereichen aufgeführten Restedefinitionen bzw. Erläuterungen können untereinander, also auch zwischen den jeweiligen Bereichen und Vorzugsbereichen beliebig kombiniert werden.

Bei den in den vorstehenden Formeln angegebenen Definitionen der Symbole wurden Sammelbegriffe verwendet, die allgemein repräsentativ für die folgenden Substituenten stehen:
Alkyl: gesättigte, geradkettige oder verzweigte Kohlenwasserstoffreste mit 1 bis 8 Kohlenstoffatomen, z.B. C₁-C₆-Alkyl wie Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 2,2-Dimethylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1-Ethyl-2-methylpropyl, Heptyl, Octyl.
Cycloalkyl: monocyclische, gesättigte Kohlenwasserstoffgruppen mit 3 bis 8 Kohlenstoffringgliedern, wie Cyclopropyl, Cyclobutyl, Cyclopentyl, Cyclohexyl, Cycloheptyl und Cyclooctyl.

Die hier verwendeten Verbindungen der Formel (II) sind literaturbekannt und u.A. nach den in WO 95/07022 angegebenen Methoden zugänglich.

Überraschender Weise wurde gefunden, dass die Kombination der oben genannten negativ beeinflussenden Liganden wie z.Bsp. 2,2'-Bipyridin oder Essigsäure, die Reaktion in hohem Maße positiv beeinflussen. Ein, wie aus Tarafder, M. T. H.; Fong, L. W. Orient. J. Chem. 2000, 16, 371 bekannter, mit einem basischen Liganden hergestellter Katalysator der Formel (III), der für diese Art von Anwendung neu ist, bewirkt eine saubere oxidative Spaltung zum gewünschten Omegadicarbonsäurederivate der Formel (I), wenn die Reaktion in Gegenwart von wässriger Carbonsäure stattfindet. Im Gegensatz zur allgemeinen Lehrmeinung, wie oben beschrieben, wurde gefunden, dass die oxidative Spaltung optimaler Weise bei einem pH von kleiner 2 abläuft.

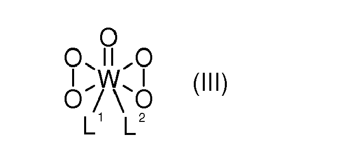

Geeignete basische Liganden L¹, L² sind monodentate *N-*Heterocyclen wie 4-*tert*-Butylpyridin, Benzimidazol, Benzoxazol, *N-*Butylimidazol, Chinolin, *iso*-Chinoline, 3-Chloropyridin, Di-*iso*propylanilin, 2-*N-*Dimethylbenzimidazol, 2-*N-*Dimethylimidazol, 2,5-Dimethylpyrrol, Indazol, 2,6-Lutidin, *N-*Methylbenzimidazol 2-Methylbenzoxazol, *N-*Methylimidazol, *N-*Methylpyrazol, 2-Phenylbenzimidazol, 4-Phenylpyridin, 2-Picolin, 3-Picolin, 4-Picolin, Pyridin, sowie die bereits beschriebenen bidentalen *N-*heterocyclische Systeme wie z.Bsp. Phenanthrolin, aus Zhu, W.; Li, H.; He, X.; Shu, H.; Yan, Y. J. Chem. Res. 2006, 12, 774. L¹, L² können gleich oder verschieden sein. Bevorzugt stehen L¹ und L² für monodentate *N-*Heterocyclen. Besonders bevorzugt stehen L¹ und L² für Pyridin.

Geeignete Carbonsäuren umfassen beispielsweise Benzoesäure, i-, n-Buttersäure, Essigsäure, Maleinsäure, Oxalsäure, Phthalsäure, Propionsäure, Terephthalsäure. Bevorzugt ist Essigsäure.

Der Katalysator wird üblicherweise in einer Menge zwischen 0,1 und 20 mol% eingesetzt, vorzugsweise zwischen 1 und 5 mol% bezogen auf die Menge an Verbindung der Formel (II).

Die oxidative Spaltung der Verbindung der Formel (II) wird durchgeführt, indem man das Substrat in ein zwischen 80 °C und 110 °C erhitztes Gemisch aus Wasser und Carbonsäure einbringt. Im Folgenden werden portionsweise 3-10 Äquivalente Wasserstoffperoxid und Katalysator über einen Zeitraum von 1 h bis 8 h zugegeben. Nach Beendigung der Reaktion innerhalb von 8 h wird das Reaktionsgemisch abgekühlt und das Produkt (I) mittels des Fachmanns bekannten Methoden, wie zum Beispiel Filtration oder Extraktion, isoliert.

### Beispiele

### Herstellung der Katalysatorenlösung:

22,4 g Wolframtrioxid Dihydrat (WO₃·2H₂O) (Freedman, M. L. J. Am. Chem. Soc. 1959, 81, 3834) werden in 224 mL wässrigem Wasserstoffperoxid (35%) suspendiert und so lange gerührt, bis man eine klare, farblose Lösung erhält. Hierzu werden 13,5 mL Pyridin gegeben. Das somit erzeugte [W(O)(O₂)₂(C₅H₅N)₂] wird direkt eingesetzt.

### Beispiel 1

### Herstellung von (3S*,4R*)-3-[(Benzyloxy)carbonyl]-4-(ethoxycarbonyl)hexandisäure

400,0 g Benzyl-ethyl-(1*S**,2*R**)-cyclohex-4-en-1,2-dicarboxylat werden in ein siedendes Gemisch aus 400 mL Wasser und 320 mL Eisessig eingeführt und im Folgenden über 3 h das 35% wässrige Wasserstoffperoxid in 9 Portionen und Katalysatorenlösung in 4 Portionen zugegeben. Das finale Reaktionsgemisch wird im Anschluss für eine weitere Stunde bei Rückfluss gekocht und dann auf 45 °C abgekühlt. Impfkristalle werden zugefügt und für 5 h bei 45 °C nachgerührt. Der ausgefallene farblose Feststoff wird abgesaugt und mit kaltem Wasser gewaschen. Man erhält 388,0 g (3*S**,4*R**)-3-[(Benzyloxy)carbonyl]-4-(ethoxycarbonyl)hexandisäure (79% der Theorie).

### Beispiel 2

### Herstellung von 3-[(Benzyloxy)carbonyl]pentandisäure

1.026 g Benzylcyclopent-3-en-1-carboxylat werden in ein siedendes Gemisch aus 1 mL Wasser und 0.8 mL Eisessig eingeführt und im Folgenden über 3 h das 35% wässrige Wasserstoffperoxid in 9 Portionen und Katalysatorenlösung in 4 Portionen zugegeben. Das finale Reaktionsgemisch wird im Anschluss für eine weitere Stunde bei Rückfluss gekocht bevor es nach Erkalten mit Ethylacetat (25 mL) verdünnt wird. Die organische Phase wird nacheinander mit wässriger Salzsäure (5%, 25 mL) und wässrigem Natrium disulfit (15%, 25 mL) gewaschen. Die organische Phase wird dann über Natriumsulfat getrocknet und unter vermindertem Druck zu einem farblosen Feststoff eingedampft. Dieser Feststoff wird mittels Chromatographie über Kieselgel aufgereinigt und man erhält 1.080 g 3-[(Benzyloxy)carbonyl]pentandisäure (80% der Theorie).

## Patentansprüche

1. Verfahren zur Herstellung von Verbindungen der Formel (I) 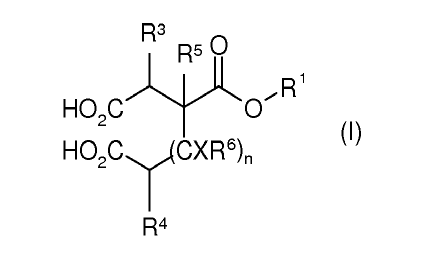 in welcher
X für COOR²,
R¹ für C₁-C₈-Alkyl, C₃-C₈-Cycloalkyl oder Benzyl,
R² für C₁-C₈-Alkyl, C₃-C₈-Cycloalkyl oder Benzyl,
R³ und R⁴ gleich oder verschieden für Wasserstoff, C₁-C₈-Alky oder C₁-C₈-Alkoxy,
R⁵ und R⁶ gleich oder verschieden für Wasserstoff oder C₁-C₈-Alky,
R³ und R⁵ oder/und R³ und R⁶ oder/und R³ und R⁴ oder/und R⁵ und R⁶ oder/und R⁵ und R⁴ oder/und R⁶ und R⁴ gemeinsam mit den Kohlenstoffatomen, an die sie gebunden sind, für gegebenenfalls durch Halogen, C₁-C₆-Alkyl, C₁-C₆-Alkoxy oder C₁-C₆-Halogenalkyl substituiertes C₃-C₅-Alkandiyl, wobei ein oder mehrere Cyclen gebildet werden können,
n für 0 oder 1;
**dadurch gekennzeichnet, dass** man Verbindungen der Formel (II) 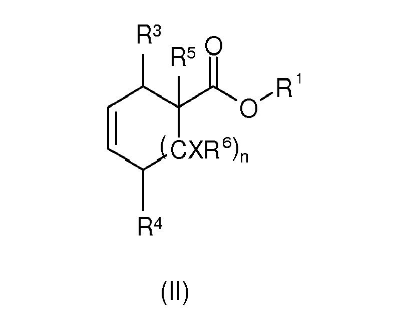 in welcher X, R¹, R³, R⁴, R⁵ und R⁶ die oben angegebenen Bedeutungen haben in Gegenwart eines Katalysators der Formel (III) 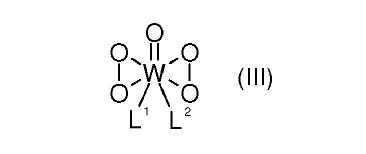 in welcher
L¹ und L² für monodentale *N-*Heterocylcen stehen oder bidentale *N-*heterocyclische Systeme bilden,
mit Wasserstoffperoxid in wässrigen Carbonsäuren umsetzt.
